# EUROPEAN PATENT SPECIFICATION

(11) **EP 4 420 521 B1**
(45) Date of publication and mention of the grant of the patent: **15.10.2025**
(21) Application number: 23158397.2
(22) Date of filing: 24.02.2023
(51) Int. Cl.: A61L 2/10, A61L 2/00, B01J 19/12, C02F 1/32, A23B 2/00, A23B 2/53, A23B 11/16, A23B 70/50

(54) **A SPACE OPTIMIZED ULTRA VIOLET GERMICIDAL IRRADIATION SYSTEM**
RAUMOPTIMIERTES UV-MIZIDES BESTRAHLUNGSSYSTEM
SYSTÈME D'IRRADIATION GERMICIDE ULTRAVIOLET OPTIMISÉ PAR L'ESPACE

(43) Date of publication of application: 28.08.2024
(73) Proprietor: Lyras DK ApS, 9000 Aalborg (DK)
(72) Inventor: KRISTENSEN, Mathias Kræmmergaard, 9000 Aalborg (DK); VESTDAM, Kasper Dalager, 9000 Aalborg (DK); MICHAELSEN, Thomas Yssing, 9000 Aalborg (DK); HØYGAARD-JØRGENSEN, Palle, 9000 Aalborg (DK)
(74) Representative: Aera A/S

(56) References cited:
- WO-A1-2019/057257
- US-A- 6 083 387

## Description

The invention relates to a UV-germicidal irradiation system for treatment of liquids, such as opaque liquids, slurries, or fermentation liquids.

### Background

Ultraviolet (UV) reactor instruments have previous been used for pasteurization of liquid food products. Examples of such instruments can be found in US 2002/096648, which discloses a reactor for irradiating ultraviolet light into a fluid reaction medium. An irradiation chamber is connected to an inlet and an outlet which allows the reaction medium to flow through the reactor while being exposed to ultraviolet light. Photo bioreactors for germicidal treatment of liquids using UV light are disclosed in US 6 083 387 A and WO 2019/057257 A1.

However, for systems designed for liquids with low ultra violet transmittance properties that require a multitude of flow strings with small individual capacities there is a need within the field for optimizing space occupied by the UV-reactor instruments to in order to achieve flows that are meaningful in an industrial production.

### Summary

Disclosed herein in a **first aspect** is a UV-germicidal irradiation system for treatment of liquids, such as opaque liquids. The UV-germicidal irradiation system comprises outer flanks including a top flank, a bottom flank, a first side flank, a second side flank, a third side flank, and a fourth side flank.

The UV-germicidal irradiation system further comprises a plurality of tubes, each of the plurality of tubes having an inlet section, a coil section, and an outlet section, wherein the coil section is a spiral-shaped tube creating a coiled fluidic pathway extending along a longitudinal axis (L) in a horizontal plane from the inlet section to the outlet section, wherein for each of the plurality of tubes, the inlet section comprises a tube inlet through which the liquid enters the tube and the outlet section comprise a tube outlet through which the liquid exits the tube, wherein the outlet section extends in parallel with the longitudinal axis (L) of the coil section such that the tube inlet and the tube outlet are positioned on the first side flank of the UV-germicidal irradiation system.

The UV-germicidal irradiation system additionally comprises one or more light sources configured to illuminate the coiled fluidic pathway of the spiral-shaped tube, wherein the one or more light sources emit light with at least one wavelength within a wavelength range between 180 nm and 315 nm.

The UV-germicidal irradiation system furthermore comprises a mounting frame comprising a plurality of openings including a plurality of light source receiving openings in which the one or more light sources are removable mounted, wherein the plurality of light source receiving openings are accessible on the first side flank of the UV-germicidal irradiation system.

The system may also be configured for slurries, and/or fermentation liquids.

By a "coiled fluidic pathway extending along a longitudinal axis (L) in a horizontal plane" is meant that the liquid flows overall horizontally through the coli section, i.e. the spiral-shaped tube, when observing over the length of the spiral-shaped tube. This means that the liquid will enter the coil section horizontally, flow through the spiral-shaped tube, and exit the coil section horizontally, hereby giving an overall horizontal flow.

One of the advantages of using light radiation as a means for cold pasteurization is that it is a very energy efficient way for partial sterilization. Pasteurization is not only limited to partial sterilization of a substance and especially a liquid at a temperature and for a time period of exposure that destroys objectionable organisms without major chemical alteration of the substance, but also covers cold pasteurization which is partial sterilization of a substance and especially a liquid in a process where heat is evaded as the main eradication of objectionable organisms without major chemical alteration of the substance. With evaded is not meant excluded but reduced. The fluidic pathway is normally designed to provide a high surface to volume ratio, increasing the exposure of light energy per unit volume with reduced self-shadowing effects from the opaque liquid being treated. In this manner it is possible to treat opaque liquids using light when the material, creating the fluidic pathway, is at least partly transparent to the radiation of light. The fluidic pathway is normally designed to provide a high surface to volume ratio, increasing the exposure of light energy per unit volume with reduced self-shadowing effects from the liquid being treated. In this manner it is possible to treat opaque liquids using light when the material, creating the fluidic pathway is at least partly transparent to the radiation of light.

It is advantageous that the floor space required around the system is minimized by using a horizontal tube configuration with inlet tube and outlet tube on the same side flank, i.e. the first side flank. Further the light sources can be removed, changed, or otherwise inspected on the same side flank as the inlet and outlet tubes can be inspected. Further, the liquid into and out of the tubes can be organized from the same side flank. Service may therefore be performed on one side only. The first side flank may be exclusively for service of the system.

On the outside surface of the side flanks, door panels may be provided for enclosing the system and possibly providing an aesthetically appealing look. Thus, in one or more examples, the UV-germicidal irradiation system comprises door panels covering one or more of the first side flank, the second side flank, the third side flank, and/or the fourth side flank. For operating the system, the door panels may be removed / pushed aside to provide access to the inside of the system, e.g. the mounting frame, the inlet and outlet tubes and the light sources. Once a user have visual access to the internal parts of the system, the light sources and the tubes may be drawn out and service operated thereupon. To provide service will normally require a total service length extending from the side flank of the system and outwards of approximately 1500 mm if the light sources has a length of 1000 mm. Being able to provide service to both the light sources and the tubes from the same side flank allows for a minimization of the service area, which is required around the system for it to be operational. In comparison, systems with inlet and outlet tubes accessible from two opposite side flanks, requires a large service area around the system as both side flanks need to be equally accessible from both side flanks.

In one or more examples, the plurality of openings in the mounting frame comprises a plurality of tube receiving openings into which the plurality of tubes can be removable mounted.

In one or more examples, the plurality of tubes are stacked on top of each other in a vertical direction in one or more vertical tube sections.

In one or more examples, the plurality of light sources are stacked on top of each other in a vertical direction in one or more vertical light source sections.

In one or more examples, the plurality of light sources are arranged in one or more vertical light source sections and the plurality of tubes are arranged in one or more vertical tube sections, wherein the vertical light source sections and the vertical tube sections are alternatingly arranged in a horizontal direction (H).

Any potential leak from the tubes will thereby be prevented from entering into the vertical light source sections.

In one or more examples, the one or more vertical light source sections and the one or more vertical tube sections are extending between the top flank and the bottom flank of the UV-germicidal irradiation system.

In one or more examples, the UV-germicidal irradiation system further comprises one or more drainage systems connected to the one or more vertical tube sections and accessible from the bottom flank and/or the first side flank of the UV-germicidal irradiation system.

In one or more examples, the vertical light source sections are separated from the vertical tube sections. The separation of the vertical light source sections and the vertical tube sections may prevent leaks due to spray/distribute by the cooling air flow if a cooling system is used. The structure separating the vertical light source sections and the vertical tube sections may incorporate and/or encompasses areas of UV transparent materials at positions that allow a significant amount of the UV radiation from the light sources to pass from the vertical light source sections into the vertical tube sections.

In one or more examples, a UV transparent material separates the vertical light source sections from the vertical tube sections. The material may also be partly UV transparent such that the transparency varies over the material.

The separation into different sections by a UV transparent material allows for a separation of the system into different thermal zones depending on the section type. As the light sources will normally submit heat to the surroundings during use, the sections with the light sources will normally be heated during use to a higher temperature than the sections with the tubes. In one or more examples, the light sources operate at a light source temperature between 0 °C and 120 °C, such as between 20 °C and 60 °C, such as between 30 °C and 50 °C.

In one or more examples, the UV-germicidal irradiation system further comprises a cooling system accessible from an outer flank different than the first side flank. Alternatively, the cooling system could be accessible from the first side flank.

In one or more examples, the cooling system is accessible from the third side flank, wherein the third side flank is positioned opposite the first side flank.

This provides for service on the tubes and the light sources on one flank and on the opposite side, service/access to the cooling element may be provided. Thereby, liquid from the tubes does not need to excess the tubes on the same side as the cooling system, e.g. a heat exchange system. Further, the service length needed for providing service to the cooling system may be significantly smaller than that required for providing service to the tubes and light sources, where a light source or a tube in its entire length may need to be removed from the system during service. A service length for providing service to the cooling system may be only 800 mm compared to the 1500 mm required for providing service to light sources or tubes having a length of 1000 mm. A reduction of the service area required around the system is thereby obtained.

In one or more examples, the cooling system is a light source cooling system for cooling the light sources possibly contained in vertical light source sections. The cooling system may be a recirculated air cooling system. The cooling system may also be an air-to-liquid heat exchange system comprising one or more cooling liquid containers containing a cooling liquid and a plurality of fans connecting the air-to-liquid heat exchange system with the plurality of light source cassettes.

In one or more examples, a space between the plurality of light sources and the spiral-shaped tube in the plurality of tubes is at least partly lined with light reflecting material, such as PTFE or polished metal, polished aluminum or steel, reflecting light emitted from the plurality of light sources.

By a reflecting material is meant that the material reflects an amount of light back. In an example, at least 70% of the light emitted onto the reflected material is reflected back. Put differently, the material may have a reflectivity of at least 70%.

In one or more examples, the UV-germicidal irradiation system further comprises a plurality of light source cassettes extending from a first end to a second end, wherein each light source cassette comprises one or more light sources illuminating the plurality of tubes. The illumination may be through the cassettes being partially transparent to UV light.

Any potential leak from the tubes will thereby stay within the vertical tube sections. The stacked configuration further provides a reduction of the space needed for the UV-germicidal irradiation system.

In one or more examples, each of the plurality of light source cassettes comprises one or more openings at the first end or at the second end for insertion and removal of the one or more light sources in the light source cassettes. The system may further comprise openings for the light cassettes such that the one or more light sources in the light source cassettes are mounted on a frame in the system.

In one or more examples, each of the plurality of light source cassettes further comprises a cassette frame with openings, wherein a first set of openings are covered by glass, e.g. quartz glass, through which light emitted from the one or more light sources can illuminate the coiled fluidic pathway of the spiral-shaped tube in the plurality of tubes.

In one or more examples, one or more filters are coated on or incorporated into the glass.

In one or more examples, the plurality of tubes are comprised in tube assemblies of two or more tubes.

In one or more examples, the tube assemblies are positioned between light source cassettes in an alternating configuration in the horizontal plane. The light sources thereby illuminate the plurality of tubes in the most efficient manner, where each light source illuminates multiple tubes and each tube are illuminated from multiple angles.

In one or more examples, the cooling system is an air-to-liquid heat exchange system comprising one or more cooling liquid containers containing a cooling liquid and a plurality of fans connecting the air-to-liquid heat exchange system with the plurality of light source cassettes, wherein the plurality of fans are configured for: 1) transferring air inside the plurality of light source cassettes, which is heated by the one or more light sources, to the air-to-liquid heat exchange system; and 2) recycling air cooled by the cooling liquid in the air-to-liquid heat exchange system to the plurality of light source cassettes.

In one or more examples, the connection between the plurality of fans and the plurality of light source cassettes is substantially airtight.

This ensures that operation of the plurality of light sources in the UV-germicidal irradiation system minimize the temperature increase of the air surrounding the UV-germicidal irradiation system.

The cooling flank has the function of removing the heat generated by the light sources. The light source cassettes are cooled by forced convection using a series of fans in parallel. The design of the light source cassettes is made practically airtight with gaskets and plates such that air is recirculated in the machine and the heat from the light sources is transferred to a cooling liquid in a series of air-to-liquid heat exchangers. The heated cooling water can be utilized at other processes, where heat is required. (Economic and environmental value). The air is recirculated in the light source cassettes, The air "circuit" is only finalized with the cassettes installed. Thus, removing the cassettes results in an open circuit.

In one or more examples, the UV-germicidal irradiation system further comprises at least one control unit. The control unit may be a unit capable of measuring and controlling e.g., flow speed, temperature, light intensity and various other properties relating to the light sources and the liquid flow through the tubes. The control unit may provide an automatic control of the system. Additionally, with a control unit, a surveillance system may be setup, so that if e.g. the pressure is decreasing, the temperature is increasing, or the light intensity is decreasing, the operator may be notified.

In one or more examples, the control unit is accessible from an outer flank different than the first side flank. The control flank may be used to operate the software of the control unit. This may require a total operating length outside the control unit of 800 mm allowing for a person to operate the control unit.

In one or more examples, the control unit is accessible from the second side flank, wherein the second side flank is positioned between and perpendicular to the first side flank and the third side flank. The first side flank may provide access for service of the tubes and the light sources, the second side flank may provide access to the control unit, and the third side flank may provide access for service of the cooling system. Thereby, the fourth side flank may be a "dead" flank, which does not provide access to any interior parts of the UV-germicidal irradiation system. The "dead" flank may be placed against a wall or another UV-germicidal irradiation system to minimize the required service area.

In one or more examples, the control unit comprises an electronic temperature monitoring module and a flow monitoring module. The control unit may also comprise an electronic temperature control module and a flow control module.

In one or more examples, the control unit automatically controls the temperature of the one or more light source and monitors a flow speed of a liquid through the fluidic pathway. Utilizing automatic control may have one of the advantages of the user saving time due to less time spend observing the system and doing manual controlled adjustments of the properties of the system. Additionally, with a control unit, a surveillance system may be setup, so that if e.g. the pressure is decreasing, the temperature is increasing, or the light intensity is decreasing, the operator may be notified. Additionally, the control unit may automatically counter the decrease in pressure, the increase in temperature, or the decrease in light intensity. Alternatively, the control unit may shut down the system if not able to counter the different irregularities.

In one or more examples, the UV-germicidal irradiation system further comprises a feed-unit comprising one or more pumps for providing the liquid to the plurality of tubes.

In one or more examples, the control unit is configured for controlling the liquid flow from the pumps and into the plurality of tubes.

In one or more examples, the feed-unit further comprises a second control unit configured for controlling the liquid flow from the pumps and into the plurality of tubes, wherein the second control unit is configured for communicating with the control unit.

In one or more examples, the UV-germicidal irradiation system further comprises a plurality of electronic cables connected to the light sources, and/or the control unit, and/or the second control unit, and/or the cooling system. The electronic cables may also connect to the pumps in the feed-unit as well as various sensors in the system.

In one or more examples, the plurality of electronic cables are accessible from an outer flank different than the first side flank.

The cable connections may be placed on top of the unit. From here they have easy access to a cable tray that runs towards the ceiling of the building in use.

In one or more examples, the plurality of electronic cables are accessible from the top flank.

In one or more examples the third side flank is positioned opposite the first side flank, and the second side flank is positioned between and perpendicular to the first side flank and the third side flank, wherein:
- the cooling system is accessible from the third side flank; and/or
- the control unit is accessible from the second side flank; and/or
- the plurality of electronic cables are accessible from the top flank; and/or
- the one or more drainage systems are accessible from the bottom flank.

In one or more examples, the UV-germicidal irradiation system further comprise a plurality of elongated support structures, wherein each spiral-shaped tube is coiled around an elongated support structure along the longitudinal axis (L) of the coiled fluidic pathway. An advantage using a support structure to coil the spiral-shaped tube around is that a support structure stabilizes the spiral-shaped tube, if said tube is e.g. made of a flexible material. The support structure may hence provide stabilization for the coil. Additionally, the support structure may have other advantage, e.g. helping with enhancing the amount of light radiated to the spiral-shaped tube by being e.g. reflective. The elongated support structures may include a solid support structure, a cylindrical mesh or anything in between. Thus in one or more examples, the plurality of support structures are made of or covered by a reflective material, such as a reflective polymeric material or a polished metal.

In one or more examples, the UV-germicidal irradiation system further comprises one or more filters positioned between the plurality of light sources and the coiled fluidic pathway of the plurality of tubes, wherein the one or more filters prevent light above a wavelength of 315 nm from reaching the coiled fluidic pathway of the plurality of tubes. By "prevent light above a wavelength of 315 nm from reaching the coiled fluidic pathway of the plurality of tubes" is meant that the one or more filters attenuates light above 315 nm by at least a factor 10, such as by at least a factor 100, such as by at least a factor 1.000, such by at least a factor 10.000. One of the advantages using one or more filter is that photo oxidation from higher wavelengths may be avoided. E.g. avoiding photo oxidation of riboflavin (around a wavelength of 446 nm) is preferred, but also avoiding photo oxidation of other components in the liquid food product, which enhances a bitter and bad flavor/taste in said food product, is preferred. Additionally, the filters may avoid hot air from contacting the coiled fluidic pathway of the plurality of tubes, hereby avoiding heating of the liquid food product.

In one or more examples, each light source cassette also comprises one or more of the one or more filters.

The coiled fluidic pathway of the plurality of tubes utilizes the flow regime occurring when the media is traveling in the fluidic pathway. The flow regime in the fluidic pathway may consists of one or several eddies, which creates a secondary flow axial on the primary flow utilizing the centrifugal force (e.g. Dean vortex flow) to enhance the surface of the liquid being exposed to UV-light emitted by the light sources. The fluid movement through the fluidic pathway may have a double vortexual pattern consistent with a Dean vortex flow. This provides an axial flow in the fluidic pathway, providing a high surface to volume ratio. This may increase the exposure of light energy per unit volume/surface area with reduced self-shadowing effects from the opaque liquid being treated. Thus, in one or more examples, a fluid movement through the coiled fluidic pathway creates a Dean Vortex flow, laminar flow, or turbulent flow in the liquid. An advantages using a Dean Vortex, laminar, or turbulent flow, in a double helix pattern, is that it may increase the exposure of light energy per unit volume/surface area with reduced self-shadowing effects from the opaque liquid being treated, hereby using less energy and time for treatment of the same volume.

In one or more examples, the spiral-shaped tube in the plurality of tubes is made of a polymeric or quartz glass material being at least ultraviolet light transparent at wavelengths between 250 and 260 nm. By a polymeric material being at least ultraviolet light transparent is meant that at least 5% of UV-light at the given wavelengths are allowed to pass through 0.1 mm of said material, i.e. in one or more examples, the polymeric material has an ultraviolet transmittance (UVT) of at least 5%, such as at least 10%, such as at least 15% at wavelengths between 250 and 260 nm, wherein the UVT is measured with a 0.1 mm path length.

In one or more examples, the spiral-shaped tube in the plurality of tubes is are made of a polymeric or quartz glass material being at least ultraviolet light transparent at wavelengths between 250 and 260 nm, such as between 240 and 270 nm, such as between 230 and 280 nm, such as between 210 and 290 nm, such as between 195 and 305 nm, such as between 180 and 315 nm.

In one or more examples, the spiral-shaped tube in the plurality of tubes made from a polymeric material selected from fluorinated ethylene propylene (FEP), polytetrafluoroethylene (PTFE), or perfluoroalkoxy alkanes (PFA) or a similar material.

In one or more examples, the spiral-shaped tube in the plurality of tubes are made from amorphous fluoropolymer (AF). Amorphous fluoropolymer (AF) is a family of amorphous fluoroplastics. These materials are similar to other amorphous polymers in optical clarity and mechanical properties, including strength. These materials are comparable to other fluoroplastics in their performance over a wide range of temperatures, in having excellent chemical resistance, and in having outstanding electrical properties. AF polymers are distinct from other fluoroplastics in that they are soluble in selected solvents, have high gas permeability, high compressibility, high creep resistance, and low thermal conductivity. AF polymers have the lowest dielectric constant of any known solid polymer. AF polymers have a low index of refraction when compared to many other known polymer.

In one or more examples, the one or more light sources are selected from a mercury-vapor light source, a xenon light source, a light emitting diode (LED), a pulsed LED, or a low pressure germicidal light source, such as a low-pressure mercury-vapor light source. A mercury-vapor light source is a gas discharge light source that uses an electric arc through vaporized mercury to produce light. The arc discharge may be confined to a small fused quartz arc tube. A xenon arc light source is a specialized type of gas discharge light source, an electric light that produces light by passing electricity through ionized xenon gas at high pressure. It produces a bright white light that closely mimics natural sunlight. A special kind of xenon light source is used in automobiles. These are actually metal-halide light sources, where a xenon arc is only used during start-up. A light emitting diode (LED) is a two-lead semiconductor light source. It is a p-n junction diode that emits light when activated. When a suitable voltage is applied to the leads, electrons are able to recombine with electron holes within the device, releasing energy in the form of photons. This effect is called electroluminescence, and the color of the light (corresponding to the energy of the photon) is determined by the energy band gap of the semiconductor. LEDs are typically small (less than 1 mm) and integrated optical components may be used to shape the radiation pattern. In one or more examples, the light sources are a metal-halide light source. A metal-halide light source is an electrical light source that produces light by an electric arc through a gaseous mixture of vaporized mercury and metal halides. It is a type of high-intensity gas discharge light source. They are similar to mercury-vapor light sources, but contain additional metal halide compounds in the quartz arc tube, which may improve the efficiency and color rendition of the light.

In one or more examples, the one or more filters are selected from bandpass filters, notch filters, or a combination of both.

In one or more examples, the UV-germicidal irradiation system is configured for inactivating or reducing a biological contaminant by an order of at least 2-Log₁₀, such as at least 3-Log₁₀, such as at least 4-Log₁₀, such as at least 5-Log₁₀, such as at least 6-Log₁₀.

In one or more examples, the biological contaminant is selected from Campylobacter jejuni, Shigella, Coxiella burnetii, Escherichia coli, Listeria monocytogenes, Mycobacterium bovis, Mycobacterium tuberculosis, Mycobacterium paratuberculosis, Salmonella spp., Yersinia enterocolitica, Brucella spp., Staphylococcus spp., Lactobacillus casei, Mycobacterium avium subspecies, Staphylococcus aureus, Streptococcus spp., Enterococcus spp., or Entrerobacter spp..

Disclosed herein in a **second aspect** is the use of a UV-germicidal irradiation system as described above for germicidal treatment of liquids, such as opaque liquids.

Disclosed herein in a **third aspect** is the use of a UV-germicidal irradiation system as described above for germicidal treatment of industrial fermentation liquids and/or fermentation broth.

Disclosed herein in a **fourth aspect** is the use of a UV-germicidal irradiation system as described above for germicidal treatment of opaque liquids, such as raw milk, milk, whey, juice, coffee, tea, soya, soylent, soda or soft drink, broth, soup, beer, smoothies, protein shake, liquid meal-replacement, cream, wine, mayonnaise, ketchup, syrup, honey, processing water, blood or blood plasma, cider, brine solution, human milk, ice tea, or combinations thereof..

Disclosed herein in a **fifth aspect** is the use of a UV-germicidal irradiation system as described above for germicidal treatment of liquids having an ultraviolet transmittance (UVT) between 1% and 90%, wherein UVT is measured at 254 nm light and 0.1 mm path length.

Disclosed herein in a **sixth aspect** is the use of a UV-germicidal irradiation system as described above for killing microorganisms in liquids, such as bacteria, mold, spores, protozoa or virus.

Disclosed herein in a **seventh aspect** is a method of germicidal treatment of a liquid. The method comprises the steps of:
- providing a liquid to be treated;
- passing the liquid to be treated through at least one tube in a UV-germicidal irradiation system as described above at a predefined flow speed, while illuminating the coiled fluidic pathway of the spiral-shaped tube with at least one wavelength within a wavelength range between 180 nm and 315 nm from the one or more light sources at a predefined power-output to obtain a germicidal treated liquid; and
- collecting the germicidal treated liquid.

The liquid may be a fermentation liquid, such as an industrial fermentation liquid and/or fermentation broth. Alternatively, or in addition, the liquid may be an opaque liquid, such as raw milk, milk, whey, juice, coffee, tea, soya, soylent, soda or soft drink, broth, soup, beer, smoothies, protein shake, liquid meal-replacement, cream, wine, mayonnaise, ketchup, syrup, honey, processing water, blood or blood plasma, cider, brine solution, human milk, ice tea, or combinations thereof.

The method may further comprise dimensioning the UV-germicidal irradiation system prior to passing the liquid to be treated through the at least one tube in the UV-germicidal irradiation system.

Dimensioning the UV-germicidal irradiation system may include selecting an optimum size of the spiral-shaped tube, and/or adjusting a flow regime of the liquid in the coiled fluidic pathway of the inside the spiral-shaped tube, and/or adjusting a light source intensity. The liquid food product flows through the spiral-shaped tube with a flow rate. In one or more examples, the flow rate measured in millilitres per minutes is between 200-6.000 ml/min, or between 500-4.000 ml/min, or between 800-2.000 ml/min, or between 900-1.100 ml/min. The predefined flow speed may be a flow speed between 1 m/s and 10 m/s, such as between 3 m/s and 8 m/s, such as between 5 m/s and 7 m/s. Flow velocity may be defined as the mass flow divided by fluid density and internal cross sectional area of the coil. Thus, the flow velocity is the vector field that is used to describe fluid motion in a mathematical manner. The entire length of the flow velocity is referred to as the flow speed. Flow velocity in fluids is the vector field that provides the velocity of fluids at a certain time and position. Flow velocity is also known as macroscopic velocity.

The fermentation liquid to be treated may be a liquid having an ultraviolet transmittance (UVT) between 5% and 90%, wherein UVT is measured at 254 nm light and 0.1 mm path length.

The predefined power-output may be a power-output of the intensity of the light sources between 100 and 1200 W/m².

### Brief description of the drawings

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example needs not have all the aspects or advantages shown. An aspect or an advantage described in conjunction with a particular example is not necessarily limited to that example and can be practiced in any other examples even if not so illustrated, or if not so explicitly described.
Figure 1 shows a UV-germicidal irradiation system in a perspective view (top of the figure) and an enlargement view of the system (bottom of the figure).
Figure 2 shows the UV-germicidal irradiation system in a view from a first side flank (top of the figure) and an enlargement view of the system (bottom of the figure).
Figure 3 shows the UV-germicidal irradiation system in a view from a second side flank (top of the figure) and an enlargement view of the system (bottom of the figure).
Figure 4 shows the UV-germicidal irradiation system in a view from a second side flank.
Figure 5 shows the UV-germicidal irradiation system in a top-down view positioned in a room surrounded by a wall.
Figure 6 shows a light source in a side view.
Figure 7 shows a tube in a side view.
Figure 8 shows a cut-through of the tube.

### Description of drawings

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

In the drawings, thicknesses of a plurality of layers and areas are illustrated in an enlarged manner for clarity and ease of description thereof. When a layer, area, element, or plate is referred to as being "on" another layer, area, element, or plate, it may be directly on the other layer, area, element, or plate, or intervening layers, areas, elements, or plates may be present therebetween. Conversely, when a layer, area, element, or plate is referred to as being "directly on" another layer, area, element, or plate, there are no intervening layers, areas, elements, or plates therebetween. Further when a layer, area, element, or plate is referred to as being "below" another layer, area, element, or plate, it may be directly below the other layer, area, element, or plate, or intervening layers, areas, elements, or plates may be present therebetween. Conversely, when a layer, area, element, or plate is referred to as being "directly below" another layer, area, element, or plate, there are no intervening layers, areas, elements, or plates therebetween.

The spatially relative terms "lower" or "bottom" and "upper" or "top", "below", "beneath", "less", "above", and the like, may be used herein for ease of description to describe the relationship between one element or component and another element or component as illustrated in the drawings. It will be understood that the spatially relative terms are intended to encompass different orientations of the device in use or operation, in addition to the orientation depicted in the drawings. For example, in the case where a device illustrated in the drawings is turned over, elements described as being on the "lower" side of other elements, or "below" or "beneath" another element would then be oriented on "upper" sides of the other elements, or "above" another element. Accordingly, the illustrative term "below" or "beneath" may include both the "lower" and "upper" orientation positions, depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below, and thus the spatially relative terms may be interpreted differently depending on the orientations described.

Throughout the specification, when an element is referred to as being "connected" to another element, the element is "directly connected" to the other element, or "electrically connected" to the other element with one or more intervening elements interposed therebetween.

The terminology used herein is for the purpose of describing particular examples only and is not intended to be limiting. As used herein, the singular forms "a," "an," and "the" are intended to include the plural forms, including "at least one," unless the content clearly indicates otherwise. "At least one" is not to be construed as limiting "a" or "an." It will be further understood that the terms "comprises," "comprising," "includes" and/or "including," when used in this specification, specify the presence of stated features, integers, steps, operations, elements, and/or components, but do not preclude the presence or addition of one or more other features, integers, steps, operations, elements, components, and/or groups thereof.

It will be understood that, although the terms "first," "second," "third," and the like may be used herein to describe various elements, these elements should not be limited by these terms. These terms are only used to distinguish one element from another element. Thus, "a first element" discussed below could be termed "a second element" or "a third element," and "a second element" and "a third element" may be termed likewise without departing from the teachings herein.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

Exemplary examples are described herein with reference to cross section illustrations that are schematic illustrations of idealized examples, wherein like reference numerals refer to like elements throughout the specification. As such, variations from the shapes of the illustrations as a result, for example, of manufacturing techniques and/or tolerances, are to be expected. Thus, examples described herein should not be construed as limited to the particular shapes of regions as illustrated herein but are to include deviations in shapes that result, for example, from manufacturing. For example, a region illustrated or described as flat may have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims. Some of the parts which are not associated with the description may not be provided in order to specifically describe exemplary examples of the present disclosure.

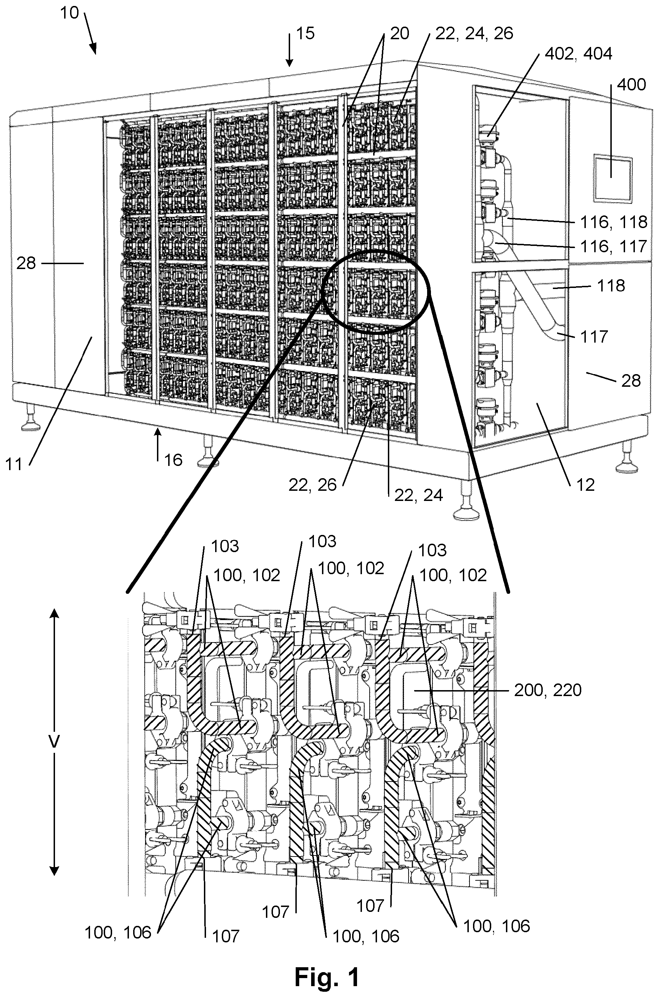

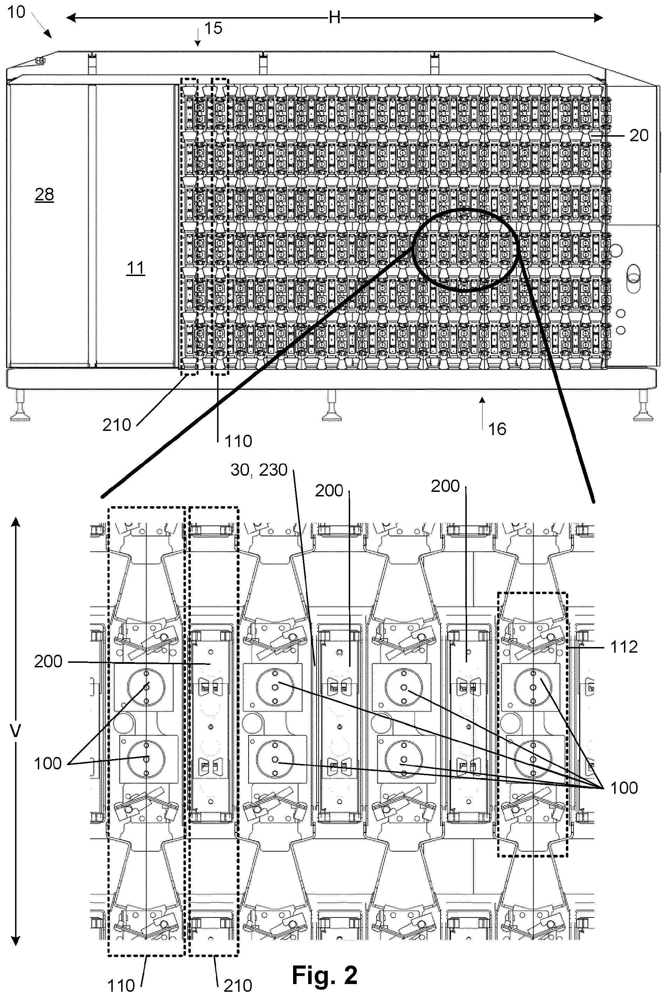

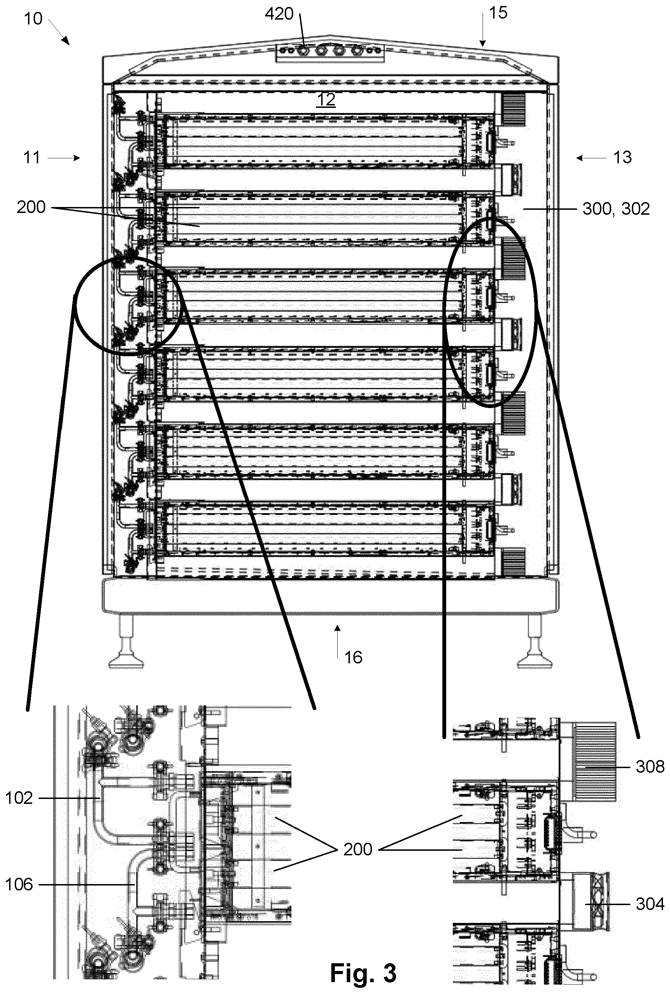

Figure 1 shows a UV-germicidal irradiation system 10 in a perspective view, figure 2 shows the system 10 in a view from a first side flank 11, and figures 3-4 show the system 10 in a view from a second side flank 12. In figures 1-3, enlargement views are also enclosed at below the full views of the system 10.

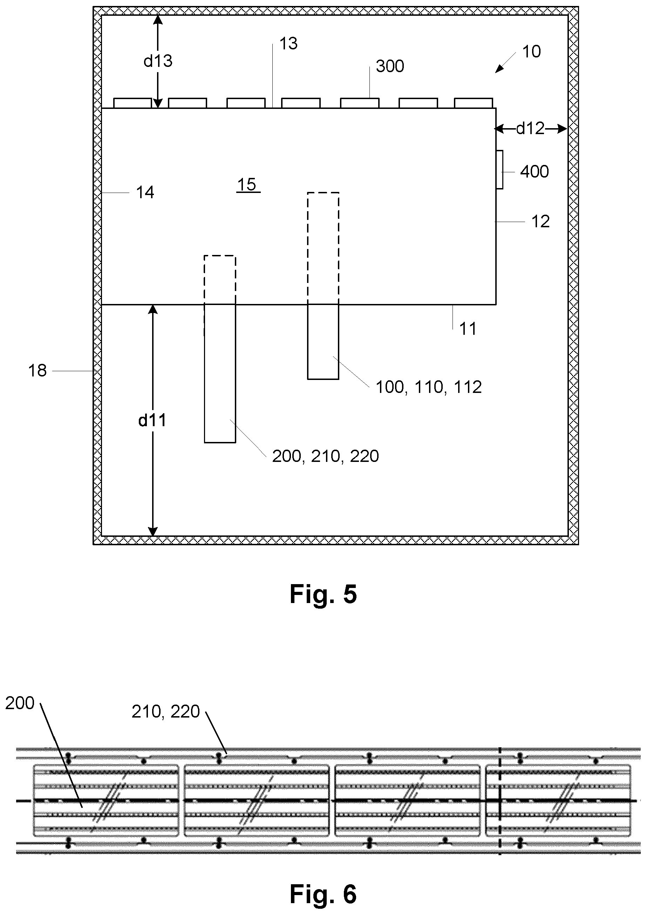

The system 10 comprises a number of outer flanks including a top flank 15, a bottom flank 16, and four side flanks including the first side flank 11, the second side flank 12 a third side flank 13, and a fourth side flank 14. The fourth side flank 14 is seen most clearly in figure 5 showing a top-down view of the system 10 positioned in a room surrounded by a wall 18.

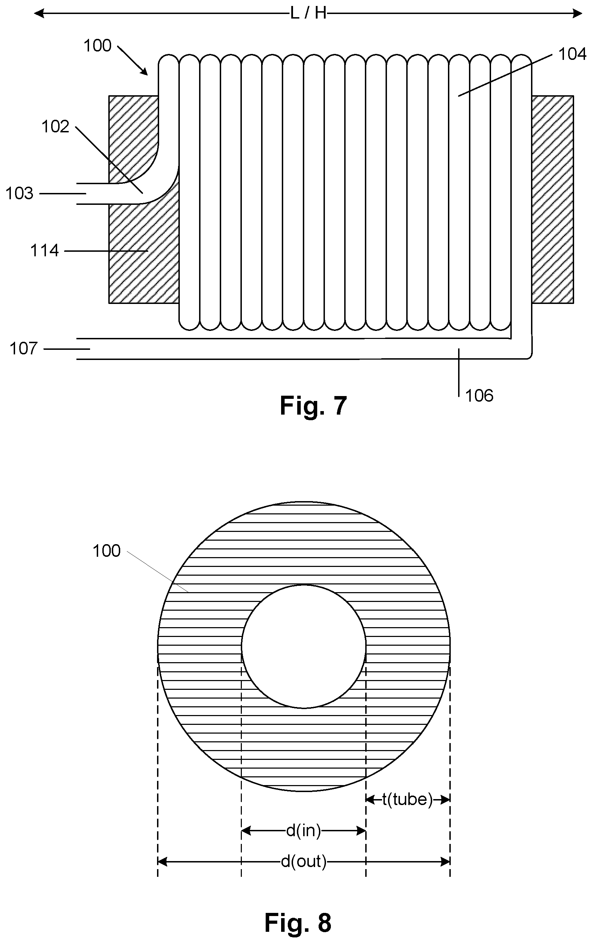

The system 10 comprises a plurality of tubes 100. As shown most clearly in figure 7, each tube 100 has an inlet section 102, a coil section 104, and an outlet section 106. The coil section 104 is a spiral-shaped tube creating a coiled fluidic pathway extending along a longitudinal axis L in a horizontal plane H from the inlet section 102 to the outlet section 106. When reference is made to spiral-shaped tube 104, it is also understood to refer interchangeably to the coil section 104 of the tube 100.

The spiral-shaped tube have an inner tube diameter d(in), an outer tube diameter d(out) and a tube thickness t(tube) as illustrated in figure 8. The inner tube diameter d(in) may be between 2 mm and 20 mm, such as between 2 mm and 18 mm, such as between 2 mm and 16 mm, such between 2 mm and 12 mm, such as between 4 mm and 12 mm, such as between 5 mm and 12 mm, or such as between 5 mm and 10 mm.

The spiral-shaped tube may have an outer tube diameter d(out) between 2.0 mm and 26.0 mm, such as between 3.0 mm and 26.0 mm, such as between 4.0 mm and 26.0 mm, such as between 5.0 mm and 26.0 mm, such as between 6.0 mm and 26.0 mm, such as between 6.0 mm and 20.0 mm, such as between 6.0 mm and 17.0 mm, such as between 6.0 mm and 14.0 mm, or such as between 6.0 mm and 12.0 mm.

In one or more examples, the spiral-shaped tube has a wall thickness t(tube) between 5% and 15% of the internal diameter d(in), such as between 5% and 14% of the internal diameter d(in), such as between 5% and 12% of the internal diameter d(in), or such as between 5% and 10% of the internal diameter d(in). The wall thickness may also be defined as the outer tube diameter minus the inner tube diameter.

The wall thickness of the one or more tubes depends on the transmission percentage of the light emitted by the one or more light sources. The higher the transmission percentage, the thicker the walls can be made.

The size of the inner diameter d(in), the outer diameter d(out) and thereby the tube thickness t(tube) is a tradeoff between the amounts of liquid capable of being treated over a given time versus the exposure of light energy per unit volume/surface area. The larger the tube is, the more liquid can pass over any given time. However, the larger the tube is, the smaller (relatively seen) the exposed area of the contained liquid volume may be.

The spiral-shaped tube 104 may have a pitch defined as the distance from center to center of the spiral-shaped tube 104 after one turn/coil of the spiral-shaped tube. In one or more examples, the spiral-shaped tube has a pitch between 1 time the outer tube diameter d(out) and 4 times the outer tube diameter d(out), such as between 1 time the outer tube diameter d(out) and 3 times the outer tube diameter d(out), or such as between 1 time the outer tube diameter d(out) and 2 times the outer tube diameter d(out). In one or more examples, the spiral-shaped tube has a pitch between 2 mm and 8 mm, such as between 3 mm and 7 mm, such as between 4 mm and 7 mm, such as between 5 mm and 7 mm, such as between 6 mm and 7 mm.

In one or more examples, the spiral-shaped tube has a coil angle measured between the spiral-shaped tube and a straight direction compared to the inlet end to the outlet end creating the fluidic pathway. The coil angle may be between 1° and 6°, such as between 2° and 5°, such as between 2° and 4°, such as between 2° and 3°.

In one or more examples, the spiral-shaped tube 104 have a compressed length from the inlet end to the outlet end between 100 mm and 1250 mm. In one or more examples, the spiral-shaped tube has a compressed length equal to or less than 1250 mm, such as equal to or less than 1150 mm, or such as equal to or less than 1050 mm. The compressed length is the length of the spiral-shaped tube 104 as shaped in the UV-germicidal irradiation system without pulling or pressing on the spiral-shaped tube 104 creating the coiled fluidic pathway measured along the longitudinal axis of the spiral-shaped tube without pulling or pressing on the spiral-shaped tube. The compressed length may also be defined as the longitudinal linear length between the inlet section to the outlet section corresponding to the coil section. The spiral-shaped tube 104 may have an extension/free length between 5 m and 20 m. The extension/free length is the total length of spiral-shaped tube. The total length is equal to the total distances one liquid unit has to pass through the spiral-shaped tube.

The inlet section 102 comprises a tube inlet 103 through which the liquid enters the tube 100 and the outlet section 106 comprise a tube outlet 107 through which the liquid exits the tube 100. In figure 1, an example is shown where two tubes 100 share a part of the inlet section 102 and likewise share a part of the outlet section 106. The tubes 100 may therefore be seen as being comprised in tube assemblies 112 of two tubes 100 as marked in figure 2.

As shown in figure 7, the outlet section 106 extends in parallel with the longitudinal axis L of the coil section 104 such that the tube inlet 103 and the tube outlet 107 are positioned on the side. As shown in figure 1, the side where inlet 103 and outlet 107 are positioned is the first side flank 11 of the UV-germicidal irradiation system 10. The floor space required around the system 10 for connecting the tube inlets 103 and tube outlets 107, conducting maintenance on the tubes 100, and for potential replacement of the tubes 100, is minimized by using a horizontal tube configuration with inlet tube 103 and outlet tube 107 on the same side flank, i.e. the first side flank 11.

The fluid movement of the liquid through the spiral-shaped tube 104 may create a Dean Vortex flow, laminar flow, or turbulent flow in the liquid.

The spiral-shaped tube 104 may be made of a material selected from the group of fluorinated ethylene propylene FEP, polytetrafluoroethylene PTFE, or perfluoroalkoxy alkanes PFA or a similar material. The spiral-shaped tube 104 may for example be from amorphous fluoropolymer AF.

The inlet section 102 and the outlet section 106 may be of the same material as the coil section 104. Alternatively, the inlet section 102 and/or the outlet section 106 may be made of a different material than the coil section 104.

The spiral-shaped tube 104 may be made of a polymeric or quartz glass material being at least ultraviolet light transparent at wavelengths between 250 and 260 nm. The ultraviolet light transparency may also cover wavelengths between 240-270 nm, such as between 230 and 280 nm, such as between 210 and 290 nm, such as between 195 and 305 nm, such as between 180 and 315 nm. By a polymeric material being at least ultraviolet light transparent is meant that at least 5% of UV-light at the given wavelengths are allowed to pass through 0.1 mm of said material. In one or more examples, the polymeric material has an ultraviolet transmittance (UVT) of at least 5%, such as at least 10%, such as at least 15% at wavelengths between 250 and 260 nm.

The system 10 may comprise elongated support structures 114, wherein each spiral-shaped tube 104 is coiled around an elongated support structure along the longitudinal axis L of the spiral-shaped tube 104 as illustrated in figure 7. The support structures 114 may be made of or covered by a reflective material, such as a reflective polymeric material or a polished metal.

The tubes 100 may be stacked on top of each other in a vertical direction (V) in a number of vertical tube sections 110 as shown most clearly in figure 2. Having the tubes 100 arranged on top of each other in the vertical direction ensures that any potential leak from the tubes 100 will thereby stay within the vertical tube section 110 which the tube 100 is positioned in.

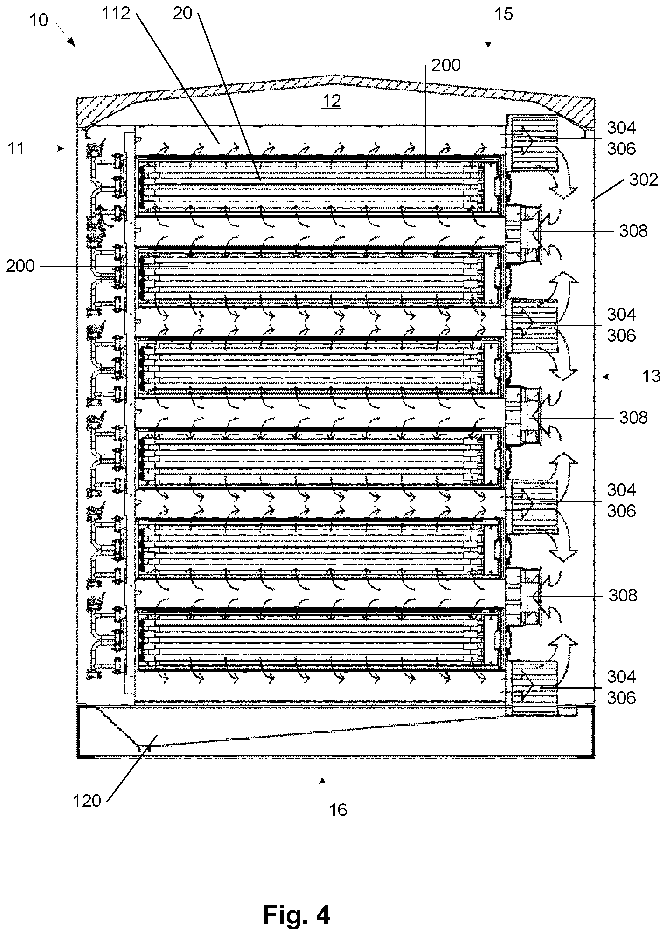

The system 10 may also comprise drainage system(s) 120 connected to the vertical tube sections 110. The drainage system(s) will normally be accessible from the bottom flank 16 and/or the first side flank 11 of the UV-germicidal irradiation system 10. Figure 4 marks the position of a drainage system 120.

The system 10 further comprises a number of light sources 200 configured to illuminate the coiled fluidic pathway of the spiral-shaped tube 104. An example of one of the light sources 200 is shown in figure 6 in a side view. The light sources 200 emit light with at least one wavelength within a wavelength range between 180 nm and 315 nm. This wavelength range is also referred to as UV-C light (approximately 100-280 nm) and UV-B light (approximately 280-315 nm). Normally, light within the UV-C wavelength range will be the preferred wavelength range.

The light source 200 may be selected from a mercury-vapor light source, a xenon light source, a light emitting diode LED, or a pulsed LED, or a low pressure germicidal light source, such as a low-pressure mercury-vapor light source.

One or more filters may be positioned between the light sources 200 and the spiral-shaped tube 104 in the plurality of tubes 100. The one or more filters 230 may prevent light above a wavelength of 315 nm from reaching the one or more spiral-shaped tube 104. By *"prevent light above* a *wavelength* of 315 *nm from reaching the one or more spiral-shaped tube"* is meant that the one or more filters attenuates light above 315 nm by at least a factor 10, such as by at least a factor 1.000, such by at least a factor 10.000. The one or more filters may be selected from bandpass filters, notch filters, or a combination of both.

The system 10 comprises a mounting frame 20 with a plurality of openings 22 between the frame parts. The openings 22 includes light source receiving openings 24 in which the light sources 200 are removable mounted. The openings 22 in the mounting frame 20 also comprise tube receiving openings 26 into which the plurality of tubes 100 can be removable mounted. As seen in figure 1, the light source receiving openings 24 and the tube receiving openings 26 are accessible on the first side flank 11 of the UV-germicidal irradiation system 10. The light source receiving openings 24 and the tube receiving openings 26 may be the same openings through which both light sources 200 and tubes 100 are accessible.

By having the light sources 200 accessible from the first side flank 11, the light sources 200 can be removed, changed, or otherwise inspected on the same side flank as the inlet tubes 103 and outlet tubes 107 of the tube 100 are inspected. Service may therefore be performed on one side flank only. The first side flank 11 may therefore be exclusively for service of the light sources 200 and the tubes 100 in the system 10.

The UV-germicidal irradiation system 10 normally comprises door panels 28 covering the side flanks 11, 12, 13, 14. The door panels 28 may be provided for enclosing the system 10 e.g. for hygienic reasons and possibly for providing an aesthetically appealing look. For operating the system 10 and performing service on the light sources 200 and the tubes 100, the door panels 28 will normally need to be removed / pushed aside to provide access to the inside of the system. In figures 1-4, the door panels 28 have been partly omitted to provide the view into the system 10 such that part of the light sources 200 and the tubes 100 are visible. Once a user have visual access to the internal parts of the system 10, the light sources 200 and the tubes 100 may be drawn out and service operated thereupon. To provide service will normally require a total service length extending from the side flank of the system and outwards a length at least corresponding to the total length of the light sources 200 and/or the tubes 100. To ensure that the light sources 200 and/or the tubes 100 can be safely removed often an additional service distance is required. If the light sources 200 and/or the tubes 100 have a total length of 1000 mm, approximately 1500 mm is needed in service area length on the first side flank. The required service distance at the first side flank is marked as d11 in figure 5. Being able to provide service to both the light sources 200 and the tubes 100 from the same side flank, i.e. the first side flank 11, allows for a minimization of the service area in comparison with standard systems where tubes and light sources are accessible from two opposite side flanks thereby requiring a large service area around the system as both side flanks need to be equally accessible from both side flanks. In figure 5, the system 10 is shown with a light source 200 / a vertical light source section 210 / a light source cassette 220 partly removed from the system 10. Likewise, in figure 5, the system 10 is shown with a tube 100 / a vertical tube section 110 / a tube assembly 112 partly removed from the system 10.

In a similar manner as the tubes 100 may be stacked on top of each other in the vertical direction V, the light sources 200 may be stacked on top of each other in a vertical direction V in vertical light source sections 210. This is illustrated in figure 2.

The light sources 200 may be arranged in light source cassettes 220 comprising one or more openings at the first end or at the second end for insertion and removal of the one or more light sources 200. The light source cassettes 220 may further comprise a cassette frame with openings possibly covered by glass, e.g. quartz glass, through which light from the light sources 200 can illuminate the spiral-shaped tube 104. One or more filters may be coated on or incorporated into the glass. Alternatively, or in addition, each light source cassette 220 may comprise one or more separate filters not included in the cassette frame.

As illustrated in figure 2, the vertical light source sections 210 and the vertical tube sections 110 may be alternatingly arranged in a horizontal direction H. The vertical light source sections 210 and the vertical tube sections 110 are normally extending between the top flank 15 and the bottom flank 16 of the UV-germicidal irradiation system 10. The vertical light source sections 210 may be separated from the vertical tube sections 110 by a UV transparent material 30 as illustrated in figure 2. The separation into different sections by a UV transparent material 30 allows for a separation of the system 10 into different thermal zones depending on the section type, i.e. whether the section contains light sources 200 or tubes 100. As the light sources 200 will normally submit heat to the surroundings during use, the sections 210 with the light sources 200 will normally be heated during use to a higher temperature than the sections 110 with the tubes 100.

A space between the light sources 200 and tubes 100 may also at least partly be lined with light reflecting material such as PTFE or polished metal, such as steel or aluminum, reflecting light from the plurality of light sources 200.

As shown in figures 3-4, the UV-germicidal irradiation system 10 comprises a cooling system 300 accessible from the third side flank 13 positioned opposite the first side flank 11.

This provides for service on the tubes 100 and the light sources 200 on one flank and on the opposite side, service/access to the cooling system 300 may be provided. Thereby, liquid from the tubes 100 will enter and excess the tubes 100 on a different side than that on which the cooling system 300 is positioned and accessible. The service length distance (illustrated as d13 in figure 5) needed for providing service to the cooling system 300 is normally significantly smaller than that required for providing service to the tubes 100 and light sources 200 (illustrated as d11 in figure 5), since a light source 200 or a tube 200 in its entire length may need to be removed from the system 10 during service. The required distance for providing service to the cooling system 300 may be only 800 mm compared to the 1500 mm required for providing service to light sources 200 or tubes 100 having a length of 1000 mm. A reduction of the service area required around the system 10 is thereby obtained.

The cooling system 300 as shown most clearly in figure 4 may be a light source cooling system 300 for cooling the light sources 200 contained in vertical light source sections 210. The cooling system 300 may be an air-to-liquid heat exchange system 302 comprising one or more cooling liquid containers 304 containing a cooling liquid 306 and a plurality of fans 308 connecting the air-to-liquid heat exchange system 302 with the plurality of light source cassettes 220. The plurality of fans 308 are configured for transferring air inside the plurality of light source cassettes 220, which is heated by the one or more light sources 200, to the one or more cooling liquid containers 304 in the air-to-liquid heat exchange system 302, and recycling air cooled by the cooling liquid 306 in the air-to-liquid heat exchange system 302 to the plurality of light source cassettes 220. The air flow is shown in figure 4.

The connection between the plurality of fans 308 and the plurality of light source cassettes 220 is normally substantially airtight. This ensures that when operation of the light sources 200 in the system 10, heat produced by the light sources 200 is only to a minor extend transferred to the air surrounding the tubes 100. Instead, the heat is transferred from the light sources 200 to the cooling air in the light source cooling system 300. Thus, it is ensured that of the heat transferred from the light sources 200 to the cooling air, a minimum is transferred to the air surrounding the tubes 100 thereby preventing undesired heating of the liquid in the tubes 100 during operation of the system 10. Air may thereby be recirculated in the system 10 and the heat from the light sources 200 may be transferred to a cooling liquid in a series of air-to-liquid heat exchangers. Thus, the cooling system 300 has the function of removing the heat generated by the light sources 200. The light sources 200 may be cooled by forced convection using a series of fans 308 in parallel. The heated cooling water may be utilized at other processes, where heat is required. This has an economic and environmental value.

The system 10 further comprises at least one control unit 400 as shown in figure 1. The control unit 400 is accessible from an outer flank different than the first side flank 11. As shown in figure 1, the control unit 400 is accessible from the second side flank 12 positioned between and perpendicular to the first side flank 11 and the third side flank 13.The second side flank 12 can therefore be seen as a control flank used to operate the software of the system 10. This service area required for operating the control unit 400 requires a total operating distance much smaller than that for operating and performing service on the tubes 100 and the light sources 200. The control flank may further incorporate a piping system 116. The piping system 116 is for distribution of the liquid to the tube inlet 103 in the plurality of tubes 100 from a main inlet pipe 117. The piping system 116 is further for distribution of the liquid from the tube outlet 107 on the plurality of tubes 100 to a main outlet pipe 118. Sensors or transmitters to monitor system operating parameters, such as volume flow, liquid pressure and liquid temperature, may also be connected to/be a part of the piping system 116 and further connected to the control unit 400 thereby, due to the close proximity of monitoring equipment to the control unit 400 to which they are connected, minimizing the installation complexity and allowing for a single point of access for installation and maintenance of monitor and control related hardware.

A service distance of 800 mm normally represents an area required to fit a person. This is illustrated as d12 in figure 5. Thus, the first side flank 11 may provide access for service of the tubes 100 and the light sources 200, the second side flank 12 may provide access to the control unit 400, and the third side flank 13 may provide access for service of the cooling system 300. Thereby, the fourth side flank 14 may be a "dead" flank, which does not provide access to any interior parts of the UV-germicidal irradiation system 10. The "dead" flank may be placed against a wall as illustrated in figure 5, or another UV-germicidal irradiation system to minimize the required service area.

As shown in figure 1, the control unit 400 may comprise an electronic temperature monitoring module 402 and a flow monitoring module 404. The control unit 400 normally automatically controls the light source temperature and monitors a flow speed of a liquid through the fluidic pathway 104. The system 10 may further comprise a feed-unit comprising one or more pumps for providing the liquid to the plurality of tubes 100. Normally, the control unit 400 is configured for controlling the liquid flow from the pumps and into the plurality of tubes 100. Alternatively, the feed-unit may comprise a second control unit configured for controlling the liquid flow from the pumps and into the plurality of tubes 100, wherein the second control unit is configured for communicating with the control unit 400.

As shown in figure 3, the system 10 further comprises a plurality of electronic cables 420 connected to the light sources 200, and/or the control unit 400, and/or the second control unit, and/or the cooling system 300. The electronic cables 420 may also connect to the pumps in the feed-unit as well as various sensors 402, 404 in the system 10. The electronic cables 420 are normally accessible from an outer flank different than the first side flank 11. As shown in figure 5, the electronic cables 420 may be placed on top of the system 10, i.e. the electronic cables 420 are accessible from the top flank 15. From here the electronic cable 420 may have easy access to a cable tray that runs towards the ceiling of the building in use.

The UV-germicidal irradiation system 10 is normally configured for inactivating or reducing a biological contaminant by an order of at least 2-Log₁₀, such as at least 3-Log₁₀, such as at least 4-Log₁₀, such as at least 5-Log₁₀, such as at least 6-Log₁₀. The biological contaminant may be selected from *Campylobacter jejuni, Shigella, Coxiella burnetii, Escherichia coli, Listeria monocytogenes, Mycobacterium bovis, Mycobacterium tuberculosis, Mycobacterium paratuberculosis, Salmonella* spp., *Yersinia enterocolitica, Brucella* spp., *Staphylococcus* spp., Lactobacillus casei, Mycobacterium avium subspecies, Staphylococcus aureus, *Streptococcus* spp., *Enterococcus* spp., or *Entrerobacter* spp..

The invention is further described in the following items.
1. A UV-germicidal irradiation system (10) for treatment of liquids, such as opaque liquids, wherein the UV-germicidal irradiation system (10) comprises:
   - outer flanks including a top flank (15), a bottom flank (16), a first side flank (11), a second side flank (12), a third side flank (13), and a fourth side flank (14);
   - a plurality of tubes (100), each of the plurality of tubes (100) having an inlet section (102), a coil section (104), and an outlet section (106), wherein the coil section (104) is a spiral-shaped tube creating a coiled fluidic pathway extending along a longitudinal axis (L) in a horizontal plane from the inlet section (102) to the outlet section (106),
      wherein for each of the plurality of tubes (100), the inlet section (102) comprises a tube inlet (103) through which the liquid enters the tube (100) and the outlet section (106) comprise a tube outlet (107) through which the liquid exits the tube (100),
      wherein the outlet section (106) extends in parallel with the longitudinal axis (L) of the coil section (104) such that the tube inlet (103) and the tube outlet (107) are positioned on the first side flank (11) of the UV-germicidal irradiation system (10);
   - one or more light sources (200) configured to illuminate the coiled fluidic pathway of the spiral-shaped tube (104), wherein the one or more light sources (200) emit light with at least one wavelength within a wavelength range between 180 nm and 315 nm; and
   - a mounting frame (20) comprising a plurality of openings (22) including a plurality of light source receiving openings (24) in which the one or more light sources (200) are removable mounted, wherein the plurality of light source receiving openings (24) are accessible on the first side flank (11) of the UV-germicidal irradiation system (10).
2. The UV-germicidal irradiation system (10) according to any preceding item, wherein the plurality of openings (22) in the mounting frame (20) comprises a plurality of tube receiving openings (26) into which the plurality of tubes (100) can be removable mounted.
3. The UV-germicidal irradiation system (10) according to any preceding item, wherein the plurality of tubes (100) are stacked on top of each other in a vertical direction in one or more vertical tube sections (110).
4. The UV-germicidal irradiation system (10) according to any preceding item, wherein the plurality of light sources (200) are stacked on top of each other in a vertical direction in one or more vertical light source sections (210).
5. The UV-germicidal irradiation system (10) according to any preceding item, wherein the plurality of light sources (200) are arranged in one or more vertical light source sections (210) and the plurality of tubes (100) are arranged in one or more vertical tube sections (110), wherein the vertical light source sections (210) and the vertical tube sections (110) are alternatingly arranged in a horizontal direction (H).
6. The UV-germicidal irradiation system (10) according to item 5, wherein the one or more vertical light source sections (210) and the one or more vertical tube sections (110) are extending between the top flank (15) and the bottom flank (16) of the UV-germicidal irradiation system (10).
7. The UV-germicidal irradiation system (10) according to item 5 or 6 further comprising one or more drainage systems (120) connected to the one or more vertical tube sections (110) and accessible from the bottom flank (16) and/or the first side flank (11) of the UV-germicidal irradiation system (10).
8. The UV-germicidal irradiation system (10) according to any of the items 5-7, wherein the vertical light source sections (210) are separated from the vertical tube sections (110).
9. The UV-germicidal irradiation system (10) according to item 8, wherein a UV transparent material (30) separates the vertical light source sections (210) from the vertical tube sections (110).
10. The UV-germicidal irradiation system (10) according to any preceding item further comprising a cooling system (300) accessible from an outer flank different than the first side flank (11).
11. The UV-germicidal irradiation system (10) according to item 10, wherein the cooling system (300) is accessible from the third side flank (13), wherein the third side flank (13) is positioned opposite the first side flank (11).
12. The UV-germicidal irradiation system (10) according to item 10 or 11, wherein the cooling system (300) is a light source cooling system (300) for cooling the light sources (200) possibly contained in vertical light source sections (210).
13. The UV-germicidal irradiation system (10) according the any preceding item, wherein a space between the plurality of light sources (200) and the spiral-shaped tube (104) in the plurality of tubes (100) is at least partly lined with light reflecting material, such as PTFE or polished metal, polished aluminum or steel, reflecting light emitted from the plurality of light sources (200).
14. The UV-germicidal irradiation system (10) according to any preceding item further comprising a plurality of light source cassettes (220) extending from a first end to a second end, wherein each light source cassette (220) comprises one or more light sources (200) illuminating the plurality of tubes (100).
15. The UV-germicidal irradiation system (10) according to item 14, wherein each of the plurality of light source cassettes (220) comprises one or more openings at the first end or at the second end for insertion and removal of the one or more light sources (200) in the light source cassettes (220).
16. The UV-germicidal irradiation system (10) according to any of the items 14-15, wherein each of the plurality of light source cassettes (220) further comprises a cassette frame with openings, wherein a first set of openings are covered by glass, e.g. quartz glass, through which light emitted from the one or more light sources (200) can illuminate the coiled fluidic pathway of the spiral-shaped tube (104) in the plurality of tubes (100).
17. The UV-germicidal irradiation system (10) according to item 16, wherein the one or more filters (230) are coated on or incorporated into the glass.
18. The UV-germicidal irradiation system (10) according to any preceding item, wherein the plurality of tubes (100) are comprised in tube assemblies (112) of two or more tubes (100).
19. The UV-germicidal irradiation system (10) according to item 18 depending on item 14 or 15, wherein the tube assemblies (112) are positioned between light source cassettes (220) in an alternating configuration in the horizontal plane.
20. The UV-germicidal irradiation system (10) according to any of the items 14-19, wherein the cooling system (300) is an air-to-liquid heat exchange system (302) comprising one or more cooling liquid containers (304) containing a cooling liquid (306) and a plurality of fans (308) connecting the air-to-liquid heat exchange system (302) with the plurality of light source cassettes (220), wherein the plurality of fans (308) are configured for:
   - transferring air inside the plurality of light source cassettes (220), which is heated by the one or more light sources (200), to the air-to-liquid heat exchange system (302); and
   - recycling air cooled by the cooling liquid (306) in the air-to-liquid heat exchange system (302) to the plurality of light source cassettes (220).
21. The UV-germicidal irradiation system (10) according to item 20, wherein the connection between the plurality of fans (308) and the plurality of light source cassettes (220) is substantially airtight.
22. The UV-germicidal irradiation system (10) according to any preceding item, wherein the UV-germicidal irradiation system (10) further comprises at least one control unit (400).
23. The UV-germicidal irradiation system (10) according to item 22, wherein the control unit (400) is accessible from an outer flank different than the first side flank (11).
24. The UV-germicidal irradiation system (10) according to item 23, wherein the control unit (400) is accessible from the second side flank (12), wherein the second side flank (12) is positioned between and perpendicular to the first side flank (11) and the third side flank (13).
25. The UV-germicidal irradiation system (10) according to any of the items 22-24, wherein the control unit (400) comprises an electronic temperature monitoring module (402) and a flow monitoring module (404).
26. The UV-germicidal irradiation system (10) according to any of the items 22-25, wherein the control unit (400) automatically controls the temperature of the one or more light source and monitors a flow speed of a liquid through the fluidic pathway.
27. The UV-germicidal irradiation system (10) according to any preceding item, wherein the UV-germicidal irradiation system (10) further comprises a feed-unit comprising one or more pumps for providing the liquid to the plurality of tubes (100).
28. The UV-germicidal irradiation system (10) according to item 27 depending on any of the items 22-26, wherein the control unit (400) is configured for controlling the liquid flow from the pumps and into the plurality of tubes (100).
29. The UV-germicidal irradiation system (10) according to item 27 depending on any of the items 22-26, wherein the feed-unit further comprises a second control unit configured for controlling the liquid flow from the pumps and into the plurality of tubes (100), wherein the second control unit is configured for communicating with the control unit (400).
30. The UV-germicidal irradiation system (10) according to any preceding item, wherein the UV-germicidal irradiation system (10) further comprises a plurality of electronic cables (420) connected to the light sources (200), and/or the control unit (400), and/or the second control unit, and/or the cooling system (300).
31. The UV-germicidal irradiation system (10) according to item 30, wherein the plurality of electronic cables (420) are accessible from an outer flank different than the first side flank (11).
32. The UV-germicidal irradiation system (10) according to item 31, wherein the plurality of electronic cables (420) are accessible from the top flank (15).
33. The UV-germicidal irradiation system (10) according to any preceding item further comprising a plurality of elongated support structures (114), wherein each spiral-shaped tube (104) is coiled around an elongated support structure along the longitudinal axis (L) of the coiled fluidic pathway.
34. The UV-germicidal irradiation system (10) according to item 33, wherein the plurality of support structures (114) are made of or covered by a reflective material, such as a reflective polymeric material or a polished metal.
35. The UV-germicidal irradiation system (10) according to any preceding item, wherein the UV-germicidal irradiation system (10) further comprises one or more filters (230) positioned between the plurality of light sources (200) and the coiled fluidic pathway of the plurality of tubes (100), wherein the one or more filters (230) prevent light above a wavelength of 315 nm from reaching the coiled fluidic pathway of the plurality of tubes (100).
36. The UV-germicidal irradiation system (10) according to item 35 depending on any of the items 14-34, wherein each light source cassette (220) also comprises one or more of the one or more filters (230).
37. The UV-germicidal irradiation system (10) according to any preceding item, wherein a fluid movement through the coiled fluidic pathway creates a Dean Vortex flow, laminar flow, or turbulent flow in the liquid.
38. The UV-germicidal irradiation system (10) according to any preceding item, wherein the spiral-shaped tube (104) in the plurality of tubes (100) is made of a polymeric or quartz glass material being at least ultraviolet light transparent at wavelengths between 250 and 260 nm.
39. The UV-germicidal irradiation system (10) according to item 38, wherein the spiral-shaped tube (104) in the plurality of tubes (100) is made of a polymeric or quartz glass material being at least ultraviolet light transparent at wavelengths between 250 and 260 nm, such as between 240 and 270 nm, such as between 230 and 280 nm, such as between 210 and 290 nm, such as between 195 and 305 nm, such as between 180 and 315 nm.
40. The UV-germicidal irradiation system (10) according to item 38 or 39, wherein the spiral-shaped tube (104) in the plurality of tubes (100) is made from a polymeric material selected from fluorinated ethylene propylene (FEP), polytetrafluoroethylene (PTFE), or perfluoroalkoxy alkanes (PFA) or a similar material.
41. The UV-germicidal irradiation system (10) according to item 38 or 39, wherein the spiral-shaped tube (104) in the plurality of tubes (100) is made from amorphous fluoropolymer (AF).
42. The UV-germicidal irradiation system (10) according to any preceding item, wherein the one or more light sources (200) are selected from a mercury-vapor light source, a xenon light source, a light emitting diode (LED), or a pulsed LED.
43. The UV-germicidal irradiation system (10) according to any preceding item, wherein the one or more light sources (200) are a low pressure germicidal light source, such as a low-pressure mercury-vapor light source.
44. The UV-germicidal irradiation system (10) according to any preceding item, wherein the one or more filters (230) are selected from bandpass filters, notch filters, or a combination of both.
45. The UV-germicidal irradiation system (10) according to any preceding item, wherein the UV-germicidal irradiation system (10) comprises door panels (28) covering one or more of the first side flank (11), the second side flank (12), the third side flank (13), and/or the fourth side flank (14).
46. The UV-germicidal irradiation system (10) according to any preceding item, wherein the UV-germicidal irradiation system (10) is configured for inactivating or reducing a biological contaminant by an order of at least 2-Log₁₀, such as at least 3-Log₁₀, such as at least 4-Log₁₀, such as at least 5-Log₁₀, such as at least 6-Log₁₀.
47. The UV-germicidal irradiation system (10) according to any preceding item, wherein the biological contaminant is selected from *Campylobacter jejuni, Shigella, Coxiella burnetii, Escherichia coli, Listeria monocytogenes, Mycobacterium bovis, Mycobacterium tuberculosis, Mycobacterium paratuberculosis, Salmonella* spp., *Yersinia enterocolitica, Brucella* spp., *Staphylococcus* spp., Lactobacillus casei, Mycobacterium avium subspecies, Staphylococcus aureus, *Streptococcus* spp., *Enterococcus* spp., or *Entrerobacter* spp..
48. Use of a UV-germicidal irradiation system (10) according to any of the items 1-47 for germicidal treatment of liquids, such as opaque liquids.
49. Use of a UV-germicidal irradiation system (10) according to any of the items 1-47 for germicidal treatment of industrial fermentation liquids and/or fermentation broth.
50. Use of a UV-germicidal irradiation system (10) according to any of the items 1-47 for germicidal treatment of opaque liquids, such as raw milk, milk, whey, juice, coffee, tea, soya, soylent, soda or soft drink, broth, soup, beer, smoothies, protein shake, liquid meal-replacement, cream, wine, mayonnaise, ketchup, syrup, honey, processing water, blood or blood plasma, cider, brine solution, human milk, ice tea, or combinations thereof.
51. Use of a UV-germicidal irradiation system (10) according to any of the items 1-47 for germicidal treatment of liquids having an ultraviolet transmittance (UVT) between 1% and 90%, wherein the UVT is measured at 254 nm light and 0.1 mm path length.
52. Use of a UV-germicidal irradiation system (10) according to any of the items 1-47 for killing microorganisms in liquids, such as bacteria, mold, spores, protozoa or virus.
53. Method of germicidal treatment of a liquid, the method comprising the steps of:
   - providing a liquid to be treated;
   - passing the liquid to be treated through at least one tube in a UV-germicidal irradiation system (10) according to any of the items 1-47 at a predefined flow speed, while illuminating the coiled fluidic pathway of the spiral-shaped tube (104) with at least one wavelength within a wavelength range between 180 nm and 315 nm from the one or more light sources (200) at a predefined power-output to obtain a germicidal treated liquid; and
   - collecting the germicidal treated liquid.
54. The method of germicidal treatment according to item 53, wherein the liquid is a fermentation liquid, such as an industrial fermentation liquid and/or fermentation broth.
55. The method of germicidal treatment according to item 53, wherein the liquid is an opaque liquid, such as raw milk, milk, whey, juice, coffee, tea, soya, soylent, soda or soft drink, broth, soup, beer, smoothies, protein shake, liquid meal-replacement, cream, wine, mayonnaise, ketchup, syrup, honey, processing water, blood or blood plasma, cider, brine solution, human milk, ice tea, or combinations thereof.
56. The method of germicidal treatment according to any of the items 53-55, wherein the method further comprises dimensioning the UV-germicidal irradiation system (10) prior to passing the liquid to be treated through the at least one tube in the UV-germicidal irradiation system (10).
57. The method of germicidal treatment according to item 56, wherein dimensioning the UV-germicidal irradiation system (10) includes selecting an optimum size of the spiral-shaped tube (104), and/or adjusting a flow regime of the liquid in the coiled fluidic pathway of the inside the spiral-shaped tube (104), and/or adjusting a light source intensity.
58. The method of germicidal treatment according to any of the items 53-57, wherein the liquid to be treated is a liquid having an ultraviolet transmittance (UVT) between 5% and 90%, wherein the UVT is measured at 254 nm light and 0.1 mm path length.
59. The method of germicidal treatment according to any of the items 53-58, wherein the predefined flow speed is a flow speed between 1 m/s and 10 m/s, such as between 3 m/s and 8 m/s, such as between 5 m/s and 7 m/s.
60. The method of germicidal treatment according to any of the items 53-59, wherein the predefined power-output of the intensity of the light sources between 100 and 1200 W/m².

### References

- 10: UV-germicidal irradiation system
- 11: first side flank
- 12: second side flank
- 13: third side flank
- 14: fourth side flank
- 15: top flank
- 16: bottom flank
- 18: wall
- 20: mounting frame
- 22: opening in the mounting frame
- 24: light source receiving opening
- 26: tube receiving opening
- 28: door panel
- 30: UV transparent material
- 100: tube
- 102: inlet section
- 103: tube inlet
- 104: coil section, spiral-shaped tube
- 106: outlet section
- 107: tube outlet
- 110: vertical tube section
- 112: tube assembly
- 114: elongated support structure
- 116: piping system
- 117: main inlet pipe
- 118: main outlet pipe
- 120: drainage system
- 200: light source
- 210: vertical light source section
- 220: light source cassette
- 230: filter
- 300: cooling system
- 302: air-to-liquid heat exchange system
- 304: cooling liquid container
- 306: cooling liquid
- 308: fan
- 400: control unit
- 402: electronic temperature monitoring module
- 404: flow monitoring module
- 420: electronic cable

- L: longitudinal axis
- H: horizontal direction
- V: vertical direction
- d11: service area distance at the first side flank
- d12: service area distance at the second side flank
- d13: service area distance at the third side flank

## Claims

1. A UV-germicidal irradiation system (10) for treatment of liquids, such as opaque liquids, wherein the UV-germicidal irradiation system (10) comprises:
- outer flanks including a top flank (15), a bottom flank (16), a first side flank (11), a second side flank (12), a third side flank (13), and a fourth side flank (14);
- a plurality of tubes (100), each of the plurality of tubes (100) having an inlet section (102), a coil section (104), and an outlet section (106), wherein the coil section (104) is a spiral-shaped tube creating a coiled fluidic pathway extending along a longitudinal axis (L) in a horizontal plane from the inlet section (102) to the outlet section (106),
wherein for each of the plurality of tubes (100), the inlet section (102) comprises a tube inlet (103) through which the liquid enters the tube (100) and the outlet section (106) comprise a tube outlet (107) through which the liquid exits the tube (100),
wherein the outlet section (106) extends in parallel with the longitudinal axis (L) of the coil section (104) such that the tube inlet (103) and the tube outlet (107) are positioned on the first side flank (11) of the UV-germicidal irradiation system (10);
- one or more light sources (200) configured to illuminate the coiled fluidic pathway of the spiral-shaped tube (104), wherein the one or more light sources (200) emit light with at least one wavelength within a wavelength range between 180 nm and 315 nm; and
- a mounting frame (20) comprising a plurality of openings (22) including a plurality of light source receiving openings (24) in which the one or more light sources (200) are removable mounted, wherein the plurality of light source receiving openings (24) are accessible on the first side flank (11) of the UV-germicidal irradiation system (10).

2. The UV-germicidal irradiation system (10) according to any preceding claim, wherein the plurality of light sources (200) are arranged in one or more vertical light source sections (210) and the plurality of tubes (100) are arranged in one or more vertical tube sections (110), wherein the vertical light source sections (210) and the vertical tube sections (110) are alternatingly arranged in a horizontal direction (H).

3. The UV-germicidal irradiation system (10) according to claim 2 further comprising one or more drainage systems (120) connected to the one or more vertical tube sections (110) and accessible from the bottom flank (16) and/or the first side flank (11) of the UV-germicidal irradiation system (10).

4. The UV-germicidal irradiation system (10) according the any preceding claim, wherein a space between the plurality of light sources (200) and the spiral-shaped tube (104) in the plurality of tubes (100) is at least partly lined with light reflecting material, such as PTFE or polished metal, polished aluminum or steel, reflecting light emitted from the plurality of light sources (200).

5. The UV-germicidal irradiation system (10) according to any preceding claim, wherein the plurality of tubes (100) are comprised in tube assemblies (112) of two or more tubes (100), wherein the UV-germicidal irradiation system (10) further comprises a plurality of light source cassettes (220) extending from a first end to a second end, wherein each light source cassette (220) comprises one or more light sources (200) illuminating the plurality of tubes (100), wherein the tube assemblies (112) are positioned between light source cassettes (220) in an alternating configuration in the horizontal plane.

6. The UV-germicidal irradiation system (10) according to claim 5 further comprising a cooling system (300) accessible from an outer flank different than the first side flank (11), wherein the cooling system (300) is an air-to-liquid heat exchange system (302) comprising one or more cooling liquid containers (304) containing a cooling liquid (306) and a plurality of fans (308) connecting the air-to-liquid heat exchange system (302) with the plurality of light source cassettes (220), wherein the plurality of fans (308) are configured for:
• transferring air inside the plurality of light source cassettes (220), which is heated by the one or more light sources (200), to the air-to-liquid heat exchange system (302); and
• recycling air cooled by the cooling liquid (306) in the air-to-liquid heat exchange system (302) to the plurality of light source cassettes (220).

7. The UV-germicidal irradiation system (10) according to claim 6, wherein the connection between the plurality of fans (308) and the plurality of light source cassettes (220) is substantially airtight.

8. The UV-germicidal irradiation system (10) according to any preceding claim, wherein the UV-germicidal irradiation system (10) further comprises at least one control unit (400) accessible from an outer flank different than the first side flank (11), wherein the control unit (400) automatically controls the temperature of the one or more light source and monitors a flow speed of a liquid through the fluidic pathway.

9. The UV-germicidal irradiation system (10) according to any preceding claim, wherein the UV-germicidal irradiation system (10) further comprises a feed-unit (410) comprising one or more pumps for providing the liquid to the plurality of tubes (100).

10. The UV-germicidal irradiation system (10) according to any preceding claim, wherein the UV-germicidal irradiation system (10) further comprises a plurality of electronic cables (420) connected to the light sources (200), and/or the control unit (400), and/or the cooling system (300), wherein the plurality of electronic cables (420) are accessible from an outer flank different than the first side flank (11).

11. The UV-germicidal irradiation system (10) according to any of the claims 4-10, wherein the third side flank (13) is positioned opposite the first side flank (11), and the second side flank (12) is positioned between and perpendicular to the first side flank (11) and the third side flank (13), wherein:
• the cooling system (300) is accessible from the third side flank (13); and/or
• the control unit (400) is accessible from the second side flank (12); and/or
• the plurality of electronic cables (420) are accessible from the top flank (15); and/or
• the one or more drainage systems (120) are accessible from the bottom flank (16).

12. The UV-germicidal irradiation system (10) according to any preceding claim further comprising a plurality of elongated support structures (114), wherein each spiral-shaped tube (104) is coiled around an elongated support structure along the longitudinal axis (L) of the coiled fluidic pathway.

13. The UV-germicidal irradiation system (10) according to any preceding claim, wherein the UV-germicidal irradiation system (10) further comprises one or more filters (230) positioned between the plurality of light sources (200) and the coiled fluidic pathway of the plurality of tubes (100), wherein the one or more filters (230) prevent light above a wavelength of 315 nm from reaching the coiled fluidic pathway of the plurality of tubes (100).

14. The UV-germicidal irradiation system (10) according to any preceding claim, wherein the spiral-shaped tube (104) in the plurality of tubes (100) is made of a polymeric or quartz glass material being at least ultraviolet light transparent at wavelengths between 250 and 260 nm, such as between 240 and 270 nm, such as between 230 and 280 nm, such as between 210 and 290 nm, such as between 195 and 305 nm, such as between 180 and 315 nm.

15. Use of a UV-germicidal irradiation system (10) according to any of the claims 1-14 for killing microorganisms in liquids, such as bacteria, mold, spores, protozoa or virus.

## Patentansprüche

1. UV-keimtötendes Bestrahlungssystem (10) zur Behandlung von Flüssigkeiten, wie beispielsweise opaken Flüssigkeiten, wobei das UV-keimtötendes Bestrahlungssystem (10) umfasst:
Außenflanken, einschließlich einer oberen Flanke (15), einer unteren Flanke (16), einer ersten Seitenflanke (11), einer zweiten Seitenflanke (12), einer dritten Seitenflanke (13) und einer vierten Seitenflanke (14);
eine Vielzahl von Rohren (100), wobei jedes der Vielzahl von Rohren (100) einen Einlassabschnitt (102), einen Spiralabschnitt (104) und einen Auslassabschnitt (106) aufweist, wobei der Spiralabschnitt (104) ein spiralförmiges Rohr ist, das einen gewundenen Flüssigkeitsweg bildet, der sich entlang einer Längsachse (L) in einer horizontalen Ebene vom Einlassabschnitt (102) zum Auslassabschnitt (106) erstreckt,
wobei für jedes der Vielzahl von Rohren (100) der Einlassabschnitt (102) einen Rohreinlass (103), durch den die Flüssigkeit in das Rohr (100) eintritt, und der Auslassabschnitt (106) einen Rohrauslass (107), durch den die Flüssigkeit aus dem Rohr (100) austritt, umfasst,
wobei sich der Auslassabschnitt (106) parallel mit der Längsachse (L) des Spulenabschnitts (104) erstreckt, derart, dass der Rohreinlass (103) und der Rohrauslass (107) an der ersten Seitenflanke (11) des UV-keimtötenden Bestrahlungssystems (10) positioniert sind;
eine oder mehrere Lichtquellen (200), die so konfiguriert sind, dass sie den gewundenen Flüssigkeitsweg des spiralförmigen Rohrs (104) beleuchten, wobei die eine oder mehreren Lichtquellen (200) Licht mit zumindest einer Wellenlänge innerhalb eines Wellenlängenbereichs zwischen 180 nm und 315 nm emittieren; und einen Montagerahmen (20), der eine Vielzahl von Öffnungen (22) umfasst, einschließlich einer Vielzahl von Lichtquellenaufnahmeöffnungen (24), in denen die eine oder mehreren Lichtquellen (200) abnehmbar montiert sind, wobei die Vielzahl von Lichtquellenaufnahmeöffnungen (24) an der ersten Seitenflanke (11) des UV-keimtötenden Bestrahlungssystems (10) zugänglich sind.

2. UV-keimtötendes Bestrahlungssystem (10) nach einem der vorhergehenden Ansprüche, wobei die Vielzahl von Lichtquellen (200) in einem oder mehreren vertikalen Lichtquellenabschnitten (210) angeordnet sind und die Vielzahl von Röhren (100) in einem oder mehreren vertikalen Röhrenabschnitten (110) angeordnet sind, wobei die vertikalen Lichtquellenabschnitte (210) und die vertikalen Röhrenabschnitte (110) in einer horizontalen Richtung (H) abwechselnd angeordnet sind.

3. UV-keimtötendes Bestrahlungssystem (10) nach Anspruch 2, ferner umfassend ein oder mehrere Entwässerungssysteme (120), die mit dem einen oder den mehreren vertikalen Rohrabschnitten (110) verbunden und von der unteren Flanke (16) und/oder der ersten Seitenflanke (11) des UV-keimtötenden Bestrahlungssystems (10) zugänglich sind.

4. UV-keimtötendes Bestrahlungssystem (10) nach einem der vorhergehenden Ansprüche, wobei ein Raum zwischen der Vielzahl von Lichtquellen (200) und dem spiralförmigen Rohr (104) in der Vielzahl von Rohren (100) zumindest teilweise mit lichtreflektierendem Material ausgekleidet ist, wie beispielsweise PTFE oder poliertem Metall, poliertem Aluminium oder Stahl, das Licht von der Vielzahl von Lichtquellen (200) reflektiert.

5. UV-keimtötendes Bestrahlungssystem (10) nach einem der vorhergehenden Ansprüche, wobei die Vielzahl von Röhren (100) in Röhrenanordnungen (112) aus zwei oder mehr Röhren (100) umfasst sind, wobei das UV-keimtötende Bestrahlungssystem (10) ferner eine Vielzahl von Lichtquellenkassetten (220) umfasst, die sich von einem ersten Ende zu einem zweiten Ende erstrecken, wobei jede Lichtquellenkassette (220) eine oder mehrere Lichtquellen (200) umfasst, die die Vielzahl von Röhren (100) beleuchten, wobei die Röhrenanordnungen (112) zwischen Lichtquellenkassetten (220) in einer abwechselnden Konfiguration in der horizontalen Ebene positioniert sind.

6. UV-keimtötendes Bestrahlungssystem (10) nach Anspruch 5, ferner umfassend ein Kühlsystem (300), das von einer anderen Außenflanke als der ersten Seitenflanke (11) zugänglich ist, wobei das Kühlsystem (300) ein Luft-zu-Flüssigkeits-Wärmetauschersystem ist (302), das einen oder mehrere Kühlflüssigkeitsbehälter (304), die eine Kühlflüssigkeit (306) enthalten, und eine Vielzahl von Lüftern (308) umfasst, die das Luft-zu-Flüssigkeits-Wärmetauschersystem (302) mit der Vielzahl von Lichtquellenkassetten (220) verbinden, wobei die Vielzahl von Lüftern (308) konfiguriert ist zum:
Übertragen von Luft innerhalb der Vielzahl von Lichtquellenkassetten (220), die durch die eine oder mehreren Lichtquellen (200) erhitzt wird, an das Luft-zu-Flüssigkeits-Wärmetauschersystem (302); und
Recyceln von durch die Kühlflüssigkeit (306) im Luft-zu-Flüssigkeits-Wärmetauschersystem (302) gekühlter Luft an die Vielzahl von Lichtquellenkassetten (220).

7. UV-keimtötendes Bestrahlungssystem (10) nach Anspruch 6, wobei die Verbindung zwischen der Vielzahl von Lüftern (308) und der Vielzahl von Lichtquellenkassetten (220) im Wesentlichen luftdicht ist.

8. UV-keimtötendes Bestrahlungssystem (10) nach einem der vorhergehenden Ansprüche, wobei das UV-keimtötend Bestrahlungssystem (10) ferner zumindest eine Steuereinheit (400) umfasst, die von einer anderen Außenflanke als der ersten Seitenflanke (11) zugänglich ist, wobei die Steuereinheit (400) automatisch die Temperatur der einen oder mehreren Lichtquellen steuert und eine Strömungsgeschwindigkeit einer Flüssigkeit durch den Flüssigkeitsweg überwacht.

9. UV-keimtötendes Bestrahlungssystem (10) nach einem der vorhergehenden Ansprüche, wobei das UV-keimtötend Bestrahlungssystem (10) ferner eine Zuführeinheit (410) umfasst, die eine oder mehrere Pumpen zum Bereitstellen der Flüssigkeit für die Vielzahl von Rohren (100) umfasst.

10. UV-keimtötendes Bestrahlungssystem (10) nach einem der vorhergehenden Ansprüche, wobei das UV-keimtötende Bestrahlungssystem (10) ferner eine Vielzahl von elektronischen Kabeln (420) umfasst, die mit den Lichtquellen (200) und/oder der Steuereinheit (400) und/oder dem Kühlsystem (300) verbunden sind, wobei die Vielzahl von elektronischen Kabeln (420) von einer anderen Außenflanke als der ersten Seitenflanke (11) zugänglich ist.

11. UV-keimtötendes Bestrahlungssystem (10) nach einem der Ansprüche 4 bis 10, wobei die dritte Seitenflanke (13) gegenüber der ersten Seitenflanke (11) positioniert ist und die zweite Seitenflanke (12) zwischen und senkrecht zur ersten Seitenflanke (11) und der dritten Seitenflanke (13) positioniert ist, wobei:
das Kühlsystem (300) von der dritten Seitenflanke (13) zugänglich ist; und/oder
die Steuereinheit (400) von der zweiten Seitenflanke (12) zugänglich ist; und/oder
die Vielzahl von elektronischen Kabeln (420) von der oberen Flanke (15) zugänglich ist; und/oder
das eine oder die mehreren Entwässerungssysteme (120) von der unteren Flanke (16) aus zugänglich sind.

12. UV-keimtötendes Bestrahlungssystem (10) nach einem der vorhergehenden Ansprüche, ferner umfassend eine Vielzahl von verlängerten Stützstrukturen (114), wobei jedes spiralförmige Rohr (104) um eine verlängerte Stützstruktur entlang der Längsachse (L) des gewundenen Flüssigkeitswegs gewunden ist.

13. UV-keimtötendes Bestrahlungssystem (10) nach einem der vorhergehenden Ansprüche, wobei das UV-keimtötende Bestrahlungssystem (10) ferner einen oder mehrere Filter (230) umfasst, die zwischen der Vielzahl von Lichtquellen (200) positioniert sind und dem gewundenen Flüssigkeitsweg der Vielzahl von Rohren (100) angeordnet sind, wobei der eine oder mehreren Filter (230) verhindern, dass Licht mit einer Wellenlänge von mehr als 315 nm den gewundenen Flüssigkeitsweg der Vielzahl von Rohren (100) erreicht.

14. UV-keimtötendes Bestrahlungssystem (10) nach einem der vorhergehenden Ansprüche, wobei das spiralförmiges Rohr (104) in der Vielzahl von Rohren (100) aus einem Polymer- oder Quarzglasmaterial hergestellt ist, das zumindest für ultraviolettes Licht bei Wellenlängen zwischen 250 und 260 nm transparent ist, wie beispielsweise zwischen 240 und 270 nm, wie beispielsweise zwischen 230 und 280 nm, wie beispielsweise zwischen 210 und 290 nm, wie beispielsweise zwischen 195 und 305 nm, wie beispielsweise zwischen 180 und 315 nm.

15. Verwendung eines UV-keimtötenden Bestrahlungssystems (10) nach einem der Ansprüche 1 bis 14 zum Abtöten von Mikroorganismen in Flüssigkeiten, wie beispielsweise Bakterien, Schimmelpilzen, Sporen, Protozoen oder Viren.

## Revendications

1. Système d'irradiation germicide ultraviolet (10) pour le traitement de liquides, tels que des liquides opaques, dans lequel le système d'irradiation germicide ultraviolet (10) comprend :
- des flancs extérieurs comportant un flanc supérieur (15), un flanc inférieur (16), un premier flanc latéral (11), un deuxième flanc latéral (12), un troisième flanc latéral (13) et un quatrième flanc latéral (14) ;
- une pluralité de tubes (100), chacun de la pluralité de tubes (100) comportant une section d'entrée (102), une section de bobine (104) et une section de sortie (106), dans lequel la section de bobine (104) est un tube en forme de spirale créant un trajet fluidique enroulé se prolongeant le long d'un axe longitudinal (L) dans un plan horizontal de la section d'entrée (102) à la section de sortie (106),
dans lequel pour chacun de la pluralité de tubes (100), la section d'entrée (102) comprend une entrée de tube (103) à travers laquelle le liquide entre dans le tube (100) et la section de sortie (106) comprend une sortie de tube (107) à travers laquelle le liquide sort du tube (100),
dans lequel la section de sortie (106) se prolonge parallèlement à l'axe longitudinal (L) de la section de bobine (104) de telle sorte que l'entrée de tube (103) et la sortie de tube (107) sont positionnées sur le premier flanc latéral (11) du système d'irradiation germicide ultraviolet (10) ;
- une ou plusieurs sources lumineuses (200) configurées pour éclairer le trajet fluidique enroulé du tube en forme de spirale (104), dans lequel les une ou plusieurs sources lumineuses (200) émettent de la lumière avec au moins une longueur d'onde comprise entre 180 nm et 315 nm ; et
- un cadre de montage (20) comprenant une pluralité d'ouvertures (22) comportant une pluralité d'ouvertures de réception de sources lumineuses (24) dans lesquelles les une ou plusieurs sources lumineuses (200) sont montées de manière amovible, dans lequel la pluralité d'ouvertures de réception de sources lumineuses (24) sont accessibles sur le premier flanc latéral (11) du système d'irradiation germicide ultraviolet (10).

2. Système d'irradiation germicide ultraviolet (10) selon une quelconque revendication précédente, dans lequel la pluralité de sources lumineuses (200) sont agencées dans une ou plusieurs sections de sources lumineuses verticales (210) et la pluralité de tubes (100) sont agencés dans une ou plusieurs sections de tube verticales (110), dans lequel les sections de sources lumineuses verticales (210) et les sections de tube verticales (110) sont agencées en alternance dans une direction horizontale (H) .

3. Système d'irradiation germicide ultraviolet (10) selon la revendication 2 comprenant également un ou plusieurs systèmes de drainage (120) reliés aux une ou plusieurs sections de tube verticales (110) et accessibles à partir du flanc inférieur (16) et/ou du premier flanc latéral (11) du système d'irradiation germicide ultraviolet (10).

4. Système d'irradiation germicide ultraviolet (10) selon une quelconque revendication précédente, dans lequel un espace entre la pluralité de sources lumineuses (200) et le tube en forme de spirale (104) dans la pluralité de tubes (100) est au moins partiellement revêtu d'un matériau réfléchissant la lumière, tel que du PTFE ou du métal poli, de l'aluminium poli ou de l'acier, réfléchissant la lumière émise par la pluralité de sources lumineuses (200).

5. Système d'irradiation germicide ultraviolet (10) selon une quelconque revendication précédente, dans lequel la pluralité de tubes (100) sont compris dans des assemblages de tubes (112) de deux ou plusieurs tubes (100), dans lequel le système d'irradiation germicide ultraviolet (10) comprend également une pluralité de cassettes de sources lumineuses (220) se prolongeant à partir d'une première extrémité à une seconde extrémité, dans lequel chaque cassette de sources lumineuses (220) comprend une ou plusieurs sources lumineuses (200) éclairant la pluralité de tubes (100), dans lequel les assemblages de tubes (112) sont positionnés entre les cassettes de sources lumineuses (220) dans une configuration alternée dans le plan horizontal.

6. Système d'irradiation germicide ultraviolet (10) selon la revendication 5, comprenant également un système de refroidissement (300) accessible à partir d'un flanc extérieur différent du premier flanc latéral (11), dans lequel le système de refroidissement (300) est un système d'échange de chaleur air-liquide (302) comprenant un ou plusieurs réservoirs de liquide de refroidissement (304) contenant un liquide de refroidissement (306) et une pluralité de ventilateurs (308) reliant le système d'échange de chaleur air-liquide (302) à la pluralité de cassettes de sources lumineuses (220), dans lequel la pluralité de ventilateurs (308) est configurée pour :
• transférer l'air à l'intérieur de la pluralité de cassettes de sources lumineuses (220), qui est chauffé par les une ou plusieurs sources lumineuses (200), vers le système d'échange de chaleur air-liquide (302) ; et
• recycler l'air refroidi par le liquide de refroidissement (306) dans le système d'échange de chaleur air-liquide (302) vers la pluralité de cassettes de sources lumineuses (220).

7. Système d'irradiation germicide ultraviolet (10) selon la revendication 6, dans lequel la connexion entre la pluralité de ventilateurs (308) et la pluralité de cassettes de sources lumineuses (220) est sensiblement étanche à l'air.

8. Système d'irradiation germicide ultraviolet (10) selon une quelconque revendication précédente, dans lequel le système d'irradiation germicide ultraviolet (10) comprend également au moins une unité de commande (400) accessible à partir d'un flanc extérieur différent du premier flanc latéral (11), dans lequel l'unité de commande (400) commande automatiquement la température des une ou plusieurs sources lumineuses et surveille une vitesse d'écoulement d'un liquide à travers le trajet fluidique.

9. Système d'irradiation germicide ultraviolet (10) selon une quelconque revendication précédente, dans lequel le système d'irradiation germicide ultraviolet (10) comprend également une unité d'alimentation (410) comprenant une ou plusieurs pompes pour fournir le liquide à la pluralité de tubes (100).

10. Système d'irradiation germicide ultraviolet (10) selon une quelconque revendication précédente, dans lequel le système d'irradiation germicide ultraviolet (10) comprend également une pluralité de câbles électroniques (420) reliés aux sources lumineuses (200) et/ou à l'unité de commande (400) et/ou au système de refroidissement (300), dans lequel la pluralité de câbles électroniques (420) sont accessibles à partir d'un flanc extérieur différent du premier flanc latéral (11).

11. Système d'irradiation germicide ultraviolet (10) selon l'une quelconque des revendications 4 à 10, dans lequel le troisième flanc latéral (13) est positionné à l'opposé du premier flanc latéral (11), et le deuxième flanc latéral (12) est positionné entre et perpendiculairement au premier flanc latéral (11) et au troisième flanc latéral (13), dans lequel :
• le système de refroidissement (300) est accessible à partir du troisième flanc latéral (13) ; et/ou
• l'unité de commande (400) est accessible à partir du deuxième flanc latéral (12) ; et/ou
• la pluralité de câbles électroniques (420) sont accessibles à partir du flanc supérieur (15) ; et/ou
• les un ou plusieurs systèmes de drainage (120) sont accessibles à partir du flanc inférieur (16).

12. Système d'irradiation germicide ultraviolet (10) selon une quelconque revendication précédente, comprenant également une pluralité de structures de support allongées (114), dans lequel chaque tube en forme de spirale (104) est enroulé autour d'une structure de support allongée le long de l'axe longitudinal (L) du trajet fluidique enroulé.

13. Système d'irradiation germicide ultraviolet (10) selon une quelconque revendication précédente, dans lequel le système d'irradiation germicide ultraviolet (10) comprend également un ou plusieurs filtres (230) positionnés entre la pluralité de sources lumineuses (200) et le trajet fluidique enroulé de la pluralité de tubes (100), dans lequel les un ou plusieurs filtres (230) empêchent la lumière au-dessus d'une longueur d'onde de 315 nm d'atteindre le trajet fluidique enroulé de la pluralité de tubes (100).

14. Système d'irradiation germicide ultraviolet (10) selon une quelconque revendication précédente, dans lequel le tube en forme de spirale (104) dans la pluralité de tubes (100) est constitué d'un matériau polymère ou de verre de quartz étant au moins transparent à la lumière ultraviolette à des longueurs d'onde comprises entre 250 et 260 nm, par exemple entre 240 et 270 nm, par exemple entre 230 et 280 nm, par exemple entre 210 et 290 nm, par exemple entre 195 et 305 nm, par exemple entre 180 et 315 nm.

15. Utilisation d'un système d'irradiation germicide ultraviolet (10) selon l'une quelconque des revendications 1 à 14 pour tuer des micro-organismes dans des liquides, tels que des bactéries, de la moisissure, des spores, des protozoaires ou des virus.
